**Europäisches Patentamt**

**European Patent Office**

**Office européen des brevets**

(19)

(11) Veröffentlichungsnummer: **0 393 521**

**A1**

(12) # EUROPÄISCHE PATENTANMELDUNG

(21) Anmeldenummer: **90107069.8**

(22) Anmeldetag: **12.04.90**

(51) Int. Cl.5: **A61B 5/14, A61M 5/32**

(30) Priorität: **21.04.89 DE 3913197**

(43) Veröffentlichungstag der Anmeldung:
**24.10.90 Patentblatt 90/43**

(84) Benannte Vertragsstaaten:
**DE FR GB IT**

(71) Anmelder: **Walter Sarstedt Geräte und Verbrauchsmaterial für Medizin und Wissenschaft**

**D-5223 Nümbrecht/Rommelsdorf(DE)**

(72) Erfinder: **Sarstedt, Walter**

**D-5223 Nümbrecht(DE)**

(74) Vertreter: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund Morgan, B.Sc.(Phys.) Robert-Koch-Strasse 1 D-8000 München 22(DE)**

(54) **Blutentnahmevorrichtung.**

(57) Eine Blutentnahmevorrichtung weist ein einseitig offenes, evakuierbares Entnahmerohr (11) auf, auf das eine Verschlußkappe (12) lösbar aufsetzbar ist, welche einen parallel zur Rohrachse (13) nach vorn vorstehnden Kanülenkonus (14) trägt, der sich konzentrisch in einen einen kleineren Durchmesser als das Entnahmerohr (11) aufweisenden, zylindrischen, als nach unten offenes Rohr ausgebildeten Ansatz (15) hineinerstreckt und der aus einer nach hinten offenen, nach vorne bis auf eine Nadeldurchlaßöffnung (19) geschlossenen steifen Hülse (20) und einem dicht auf den Kanülenkonus (14) aufsetzbaren, einen Kanal (22) besitzenden Gegenstück (21) besteht. Auf den Ansatz (15) ist eine dazu komplementäre, hinten offene und vorne eine beidseitig angeschärfte Kanüle (16) tragende Führungshülse (17) axial von vorne aufschiebbar, wobei das sich in das Innere der Führungshülse erstrekkende Kanülenende (16a) eine nahe der Nadeldurchlaßöffnung (19) angeordnete, in axialer Ausrichtung mit diesem befindliche sowie einen Bestandteil des Ansatzes (15) bildende Elastikplatte (18) durchsticht und so eine Strömungsverbindung zwischen dem Inneren der Kanüle (16) und dem Inneren des Kanülenkonus (14) herstellt. Erfindungsgemäß ist das Gegenstück ein in die steife Hülse (20) eingesetzter weichelastischer Stopfen (21), welcher einen zur Schraubkappe (12) hin offenen, mit dem Kanülenkonus (14) in einen den Ansatz (15) fest, jedoch lösbar an der Schraubkappe (12) haltenden Klemmsitz bringbaren Kanal (22) aufweist, der vorn durch die Elastikplatte (18) verschlossen ist, welche mit dem Stopfen (21) aus einem Stück besteht.

Fig. 2

Die Erfindung betrifft eine Blutentnahmevorrichtung mit einem einseitig offenen, evakuierten bzw. evakuierbaren Entnahmerohr, auf das eine Verschlußkappe lösbar aufsetzbar ist, welche einen parallel zur Rohrachse nach vorn vorstehenden Kanülenkonus trägt, der sich vorzugsweise konzentrisch in einen einen kleineren Durchmesser als das Entnahmerohr aufweisenden, zumindest im wesentlichen zylindrischen, im wesentlichen als nach unten offenes Rohr ausgebildeten Ansatz hineinerstreckt, der aus einer nach hinten offenen, nach vorne bis auf eine Nadeldurchlaßöffnung geschlossenen steifen Hülse und einem dicht auf den Kanülenkonus aufsetzbaren, einen Kanal aufweisenden Gegenstück besteht und auf den eine im wesentlichen dazu komplementäre, hinten offene und vorne eine beidseitig angeschärfte Kanüle vorzugsweise ebenfalls konzentrisch tragende Führungshülse axial von vorne aufschiebbar ist, wobei das sich in das Innere der Führungshülse erstreckende Kanülenende eine nahe der Nadeldurchlaßöffnung angeordnete, in axialer Ausrichtung mit diesem befindliche sowie einen Bestandteil des Ansatzes darstellende Elastikplatte durchsticht und so eine Strömungsverbindung zwischen dem Inneren der Kanüle und dem Inneren des Kanülenkonus herstellt.

Eine derartige Blutentnahmevorrichtung ist aus den Fig. 5 und 6 der DE-C2 29 48 653 bekannt. Bei dem dort verwendeten Ansatz handelt es sich um einen Adapter, der die Verwendung der die doppelendige Kanüle tragenden Führungshülse auch in Verbindung mit einem üblichen Kanülenkonus ermöglichen soll.

Es ist jedoch nicht zweckmäßig, diesen bekannten Adapter als ständig an dem Kanülenkonus angeordneten Ansatz zu verwenden, der nur in den Ausnahmefällen, wo statt der Führungshülse eine normale Kanüle verwendet werden soll, vom Kanülenkonus abgezogen wird, um Platz für den am rückwärtigen Ende der normalen Kanüle vorgesehenen Aufnahmekonus zu schaffen. Der bekannte Adapter müßte zu diesem Zweck nämlich so fest auf den Kanülenkonus der Schraubkappe aufgedrückt werden, daß beim normalen Gebrauch unter Verwendung der kreiszylindrischen Führungshülse eine ungewollte Lösung des Adapters von der Schraubkappe nicht erfolgen kann. Dadurch würde aber der bekannte Adapter so fest auf dem Kanülenkonus sitzen, daß eine Lösung von Hand nicht oder nur unter großen Schwierigkeiten möglich ist. Das feste Aufpressen des aus steifen Kunststoffmaterial bestehenden Adapters auf den Kanülenkonus birgt auch die Gefahr in sich, daß die Oberfläche des Kanülenkonus verformt oder beschädigt wird, so daß sie ihre Funktion als Dichtfläche gegenüber dem Aufnahmekonus einer normalen Kanüle nicht mehr in der erforderlichen Weise erfüllen kann.

Es ist auch schon bekannt geworden, den zylindrischen Ansatz als ein zylindrisches Gummiteil auszubilden, welches eine an den Kanülenkonus angepaßte Bohrung aufweist und klemmend auf den Kanülenkonus aufgesetzt wird. Wegen der weichelastischen Eigenschaften von Gummi findet hierbei zwar eine gewisse dichtende Einfassung des Kanülenkonus statt, doch weitet sich beim Aufsetzen des Gummiansatzes auf den Kanülenkonus dieser nicht nur in unerwünschter Weise auf, was eine Verformung der für die Führungshülse vorgesehenen Mantelfläche zur Folge hat, sondern es ist auch die zur Halterung des Ansatzes am Kanülenkonus erforderliche Klemmkraft wegen der gummielastischen Eigenschaften des Ansatzes in unerwünschter Weise beschränkt.

Das Ziel der vorliegenden Erfindung besteht darin, eine Blutentnahmevorrichtung der eingangs genannten Gattung zu schaffen, bei der der lösbar an der Schraubkappe angeordnete Ansatz beim bestimmungsgemäßen Gebrauch mit der Führungshülse ausreichend fest und dicht am Kanülenkonus angeordnet ist, gleichwohl aber durch Aufbringung nicht zu geringer Lösekräfte von Hand vom Kanülenkonus abgezogen werden kann, um statt der eine doppelseitig angeschärfte Kanüle aufweisenden Führungshülse eine normale Kanüle mit konischem Aufnahmeraum am rückwärtigen Ende verwenden zu können, wobei die folgenden Forderungen gleichzeitig erfüllt sein sollen:

a) Absolute Dichtheit zwischen Kanülenkonus und Ansatz im Bereich des Überganges vom Kanülenende zum Eintrittsende des Kanülenkonus;

b) fester und nur durch relativ große, aber noch von Hand auszuübende Kräfte lösbarer Klemmsitz des Ansatzes auf dem Kanülenkonus;

c) Fernhaltung von steifen, die Oberfläche des Kanülenkonus verformenden und/oder beschädigenden Befestigungsflächen von der Oberfläche des Kanülenkonus und

d) Vermeidung einer Aufweitung der Mantelfläche des Ansatzes aufgrund der Preßverbindung mit dem Kanülenkonus.

Zur Lösung dieser Aufgabe sieht die Erfindung vor, daß das Gegenstück ein in die steife Hülse eingesetzter, weichelastischer Stopfen, der vorzugsweise aus Gummi besteht, ist, welcher einen zur Schraubkappe hin offenen, mit dem Kanülenkonus in einen den Ansatz fest, jedoch lösbar an der Schraubkappe haltenden Klemmsitz bringbaren Kanal aufweist, der vorn durch die Elastikplatte verschlossen ist. Bevorzugt besteht die Elastikplatte dabei mit dem Stopfen aus einem Stück.

Der Erfindungsgedanke ist also darin zu sehen, daß der Ansatz außen steif und innen weichelastisch ist, so daß durch das Aufpressen des Ansatzes auf den Kanülenkonus zwar durch entsprechende Verformung des weichelastischen Materials die erforderlichen Dicht- und Klemmkräfte für die Halterung erzielt werden, jedoch damit keine Aufweitung des Ansatzes verbunden ist, weil der Stopfen durch die steife Hülse bezüglich seiner Außenform bestimmt und festgehalten wird. Auf diese Weise werden auch wesentlich größere Klemmkräfte für die Halterung des Ansatzes am Kanülenkonus erzielt, denn beim Aufstecken des im Querschnitt kleiner als der Kanülenkonus ausgebildeten Kanals auf den Kanülenkonus wird das weichelastische Material des Stopfens lediglich in radialer Richtung komprimiert, jedoch nicht radial aufgeweitet.

Nachdem ausschließlich weichelastisches Material mit der Oberfläche des steifen Kanülenkonus in Eingriff steht, besteht keine Gefahr einer Verformung und/oder Beschädigung der Oberfläche des Kanülenkonus, so daß diese ihren einwandfreien Zustand für die gelegentliche Aufnahme eines normalen Kanülenkonus voll beibehält.

Trotz des festen Sitzes und der guten Abdichtung kann der erfindungsgemäße Ansatz durch die Aufbringung etwas stärkerer Kräfte von Hand vom Kanülenkonus gelöst werden, indem eine Drehbewegung mit einer axialen Abzugsbewegung kombiniert wird.

Nachdem der erfindungsgemäße Stopfen aus weichelastischem Material ausgebildet ist, eignet er sich auch dafür, daß er die Funktion der durchstechbaren und sich wieder von selbst schließenden Elastikplatte übernimmt. Bei dieser Ausführungsform erfüllt der erfindungsgemäße weichelastische, vorzugsweise aus Gummi bestehende Stopfen mehrere Funktionen wie folgt:
- er bildet die durchstechbare und sich wieder von selbst verschließende Elastikplatte;
- er dient der Abdichtung insbesondere des vorderen Endes des Kanülenkonus;
- er dient in Zusammenwirkung mit der steifen Hülse der Aufbringung der Klemmkraft für die Halterung des Ansatzes am Kanülenkonus.

Besonders vorteilhaft ist es, wenn die vordere Stirnfläche der Elastikplatte mit der vorderen Stirnfläche des Stopfens bündig ist. Auf diese Weise kann der vorzugsweise außen als kreiszylindrischer Block mit ebenen Stirnflächen ausgebildete Stopfen passend bis an die innen die Nadeldurchlaßöffnung aufweisende ebene Stirnwand der Hülse eingeschoben werden, wodurch die Elastikplatte beim Herausziehen der Nadel der Führungshülse nach vorn abgestützt ist.

Eventuell vorteilhaft ist es, wenn der Stopfen eine kreiszylindrische Mantelfläche und die Hülse eine kreiszylindrische Innenumfangsfläche aufweist.

Eine weitere vorteilhafte Ausgestaltung der Erfindung kennzeichnet sich dadurch, daß der Stopfen zumindest bei aufgesetztem Ansatz mit seiner Mantelfläche gegen die Innenumfangsfläche der Hülse gepreßt wird. Auf diese Weise wird durch die radiale Halterung eine besonders starke Anpreßkraft am Kanülenkonus erzielt.

Für die Montage und den späteren Gebrauch ist es zweckmäßig, wenn der Stopfen ohne eingesteckten Kanülenkonus im leichten, selbsthaltenden Reibsitz in der Hülse steckt. Der Stopfen soll also nicht von selbst aus der steifen Ansatzhülse herausfallen; es genügt, wenn die für die Abdichtung und die Halterung am Kanülenkonus erforderlichen Klemmkräfte erst beim Aufstecken des Ansatzes auf den Kanülenkonus erzielt werden.

Eine besonders vorteilhafte praktische Ausführungsform ist so ausgebildet, daß der Kanal im Bereich der Spitze des Kanülenkonus dichtend vom Material des Stopfens umgeben ist, so daß dort ein Dichtbereich vorliegt.

Bei dieser Ausführungsform ist es weiter vorteilhaft, wenn der Kanal hinter dem Dichtbereich zumindest eine axial verlaufende Entlüftungsnut aufweist und in diesem Bereich in festem Klemmsitz mit dem Kanülenkonus lösbar verbunden ist.

Erfindungsgemäß wird also klar zwischen einem in erster Linie der Dichtung dienenden Bereich am vorderen Ende des Kanülenkonus und einem in erster Linie dem Festhalten des Ansatzes am Kanülenkonus dienenden hinteren Klemmbereich unterschieden. Auf diese Weise werden die mit einer starken Klemmung verbundenen starken Verformungen des Stopfens vom eigentlichen Dichtbereich ferngehalten, wo es weniger auf übermäßige Klemmkräfte denn auf ein gleichmäßiges Anliegen von möglichst unverformten Bereichen des Stopfens am Umfang des Kanülenkonus ankommt. Wo starke elastische Verformungen erfolgen, muß immer mit einem gewissen Hängenbleiben des weichelastischen Materials bei der Montage und deswegen mit Un dichtigkeiten gerechnet werden.

Erfindungsgemäß werden in dem Bereich hinter dem Dichtbereich sogar bewußt axial verlaufende Entlüftungs-Kanäle bzw. -Nuten vorgesehen, damit es hier nicht durch Lufteinschlüsse zu unerwünschten Verformungen oder undefinierten Anlageflächen kommt. Vor allem wird auf diese Weise vermieden, daß sich Lufteinschlüsse bis in den Dichtbereich fortpflanzen und dort zu Undichtigkeiten führen. Beim Abziehen des Ansatzes vom Kanülenkonus wird so auch das Mitreißen von Flüssigkeit vermieden.

Der Grundgedanke der vorstehend angegebenen Ausführungsform ist also darin zu sehen, daß im vorderen Bereich des Kanülenkonus ein nur geringfügig klemmendes, dafür aber besonders gleichmäßiges und dichtes Anliegen des Materials des Stopfens am Umfang des Kanülenkonus gewährleistet wird, während dahinter die Dichtheit in axialer Richtung bewußt vermieden, dafür jedoch eine stark erhöhte Klemmkraft aufgebracht wird.

Da es auf eine Dichtung nur im vorderen Bereich des Kanülenkonus ankommt, sieht die Erfindung zweckmäßigerweise vor, daß sich der Dichtbereich über 5 bis 20 %, insbesondere etwa 10 % der Länge des mit dem Kanülenkonus in Kontakt stehenden Teils des Kanals erstreckt.

Eine besonders klare Trennung von Dicht- und Klemmbereich kann dadurch erzielt werden, daß der Kanal hinter der Elastikplatte einen kurzen Abschnitt runden Querschnitts aufweist, der einen geringfügig kleineren Durchmesser als der vordere Endbereich des ebenfalls einen kreisrunden Querschnitt aufweisenden Kanülenkonus besitzt und in den der vordere Endbereich des Kanülenkonus dichtend eingreift.

Dabei soll insbesondere vorgesehen sein, daß der Querschnitt des Kanals sich hinter dem Dichtbereich erweitert, jedoch nur so geringfügig, daß aufgrund der konischen Erweiterung des Kanülenkonus noch ein Klemmbereich vorliegt.

Schließlich ist es zweckmäßig, wenn sich der Querschnitt des Kanals hinter dem Dichtbereich sprungartig erweitert und dann bis zum hinteren Ende des Kanals unverändert bleibt.

Aufgrund der vorzugsweise sprungartigen Querschnittsänderung des Kanals am hinteren Ende des Dichtbereichs wird die Einwirkung von Klemmspannungen in dem hierfür besonders empfindlichen Dichtbereich wirksam vermieden.

Aufgrund der sich konisch erweiternden Ausbildung des Kanülenkonus wird auf diese Weise weiter das Auftreten von Klemmspannungen im vorderen Bereich des Kanülenkonus vermieden. Die Klemmspannungen treten aufgrund der Erweiterung des Kanülenkonus nach hinten vielmehr erst in einem deutlichen Abstand vom Dichtbereich auf, so daß Dicht- und Klemmbereich durch einen beträchtlichen axialen Abstand voneinander getrennt sind. Eine bevorzugte praktische Ausführungsform kennzeichnet sich dadurch, daß im Klemmbereich wenigstens eine, vorzugsweise jedoch mehrere über den Umfang verteilte axiale Entlüftungsnuten von solcher Tiefe vorgesehen sind, daß auch bei auf dem Kanülenkonus aufgesetztem Ansatz noch ein Luftdurchlaß am hinteren Ende verbleibt.

Eine weitere vorteilhafte Ausführungsform ist dadurch definiert, daß bei quadratischem Querschnitt des Kanals im Klemmbereich die axialen Entlüftungsnuten automatisch durch die Ecken des quadratischen Querschnitts verwirklicht werden. Die Bereiche zwischen den Ecken dienen als von vier Seiten gegen dem Kanülenkonus drückende Klemmbacken.

Das Fernhalten von zu starken Klemmspannungen im Bereich des vorderen Endes des Kanülenkonus wird weiter dadurch gefördert, daß sich im Bereich des vorderen Endes des Kanülenkonus in der Mantelfläche des Stopfens eine Umfangsnut befindet, welche ein sanftes und gleichmäßiges federndes Aufweiten des Kanals beim Einstecken der Spritze des Kanülenkonus in diesem Bereich und somit ein gleichmäßiges dichtes Anliegen des Stopfens im Dichtbereich am Umfang des Kanülenkonus gestattet.

Eine baulich besonders kompakte Anordnung ist dadurch gekennzeichnet, daß die Spitze des Kanülenkonus zumindest bis annähernd an die hintere Fläche der Elastikplatte reicht. Die Spitze kann sogar die Elastikplatte von hinten berühren, wodurch auch im Bereich der Stirnfläche des weiülenkonus eine Abdichtung erzielt wird.

Einem kompakten Aufbau dient auch die weitere Maßnahme, wonach die Umfangsnut unmittelbar hinter dem Bereich der Elastikplatte beginnt.

Besonders vorteilhaft ist es, wenn die Hülse am Ansatz und die Führungshülse Bajonettverschlußmittel aufweisen, wodurch eine Befestigung der Führungshülse am Ansatz gemäß der DE-C2 30 49 503 möglich ist. Bei dieser Ausführungsform besteht der weitere Vorteil, daß durch Anordnung der Führungshülse am Ansatz mit möglichst wenig Reibung und vorzugsweise sogar mit Spiel die Anbringung der Führungshülse am Ansatz sowie die Abnahme der Führungshülse vom Ansatz problemlos möglich ist, ohne daß die Gefahr besteht, daß der Ansatz von dem Kanülenkonus abgezogen wird. Aufgrund der Kombination eines weichelastischen Stopfens mit einer steifen Hülse wird eine Verformung des Außenumfanges des Ansatzes vermieden, die zu einer verstärkten Klemmung der Führungshülse auf dem Ansatz führen könnte.

Die Erfindung wird im folgenden beispielsweise anhand der Zeichnung beschrieben; in dieser zeigt:

Fig. 1 eine Seitenansicht einer erfindungsgemäßen Blutentnahmevorrichtung,

Fig. 2 eine vergrößerte, teilweise geschnittene Seitenansicht des vorderen Endes der Blutentnahmevorrichtung nach Fig. 1, wobei die Führungshülse 17 zunächst nur teilweise auf den Ansatz 15 aufgeschoben dargestellt ist,

Fig. 3 eine teilweise geschnittene Seitenansicht analog Fig. 2, wobei jedoch die Führungshülse 17 weiter auf den Ansatz 15 aufgeschoben ist,

Fig. 4 einen Schnitt nach Linie IV-IV in Fig. 2,

Fig. 5 einen Schnitt nach Linie V-V in Fig. 2,

Fig. 6 einen Schnitt nach Linie VI-VI in Fig. 2,

Fig. 7 einen Schnitt nach Linie V-V in Fig. 2 einer etwas abgewandelten Ausführungsform und

Fig. 8 eine teilweise geschnittene Ansicht analog Fig. 1, wobei jedoch auf den Kanülenkonus 14 statt des Ansatzes 15 eine normale Kanüle 37 aufgesetzt ist.

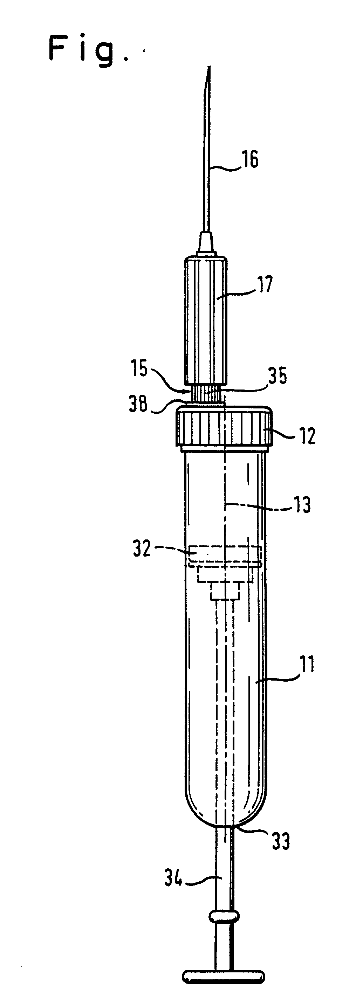

Nach Fig. 1 ist im Entnahmerohr 11 einer Blutentnahmevorrichtung ein Kolben 32 axial verschiebbar angeordnet, der mittels einer durch das hintere Ende 33 des Entnahmerohrs 11 hindurchgeführten Kolbenstange 34 axial verschoben werden kann. Das vordere Ende des Entnahmerohrs 11 ist offen und mit einem Außengewinde versehen, auf das eine mit einem Innengewinde versehene Schraubkappe 12 aufgeschraubt ist.

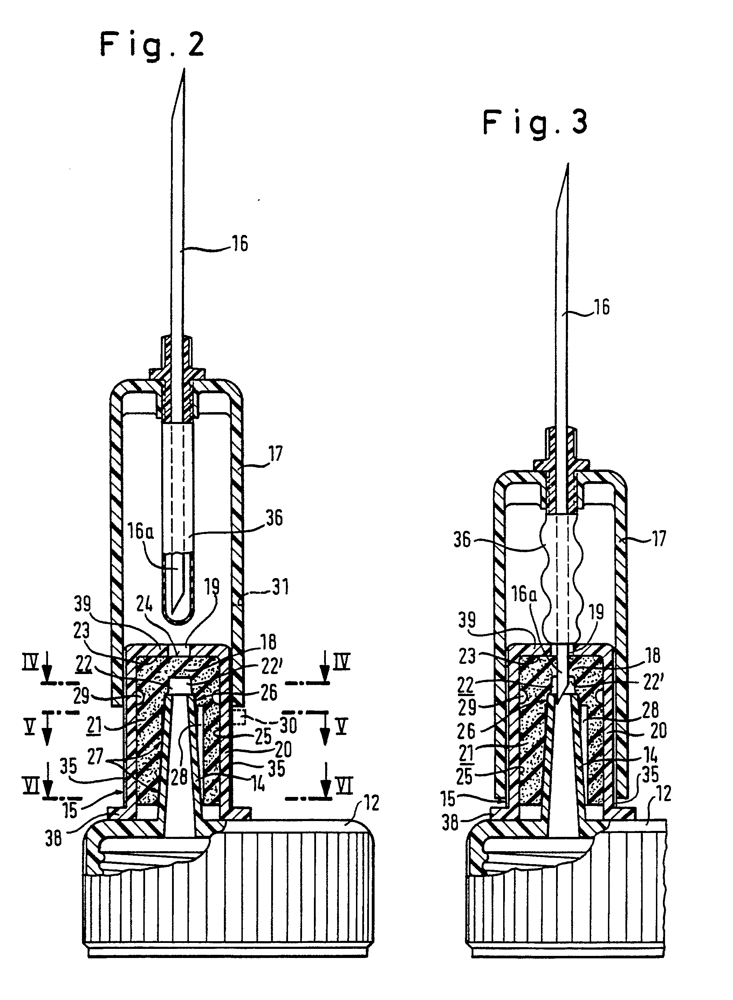

Nach Fig. 2 erstreckt sich vom vorderen Ende der Schraubkappe 12, zur Achse 13 des Entnahmerohrs 11 exzentrisch ein Kanülenkonus 14 in einen Ansatz 15 hinein, der aus einem inneren weichelastischen Stopfen 21 z.B. aus Gummi und einer diesen umgebenden steifen Hülse 20 z.B. aus Kunststoff besteht. Auf den außen mit axialen Entlüftungsrillen 35 versehenen Ansatz 15 ist eine komplementär dazu ausgebildete Führungshülse 17 aufschiebbar, in deren vorderer Stirnwand eine doppelseitig angeschärfte Kanüle 16 angeordnet ist, deren hinterer Teil 16a von einem elastischen Schlauch 36 dicht umgeben ist. In Fig. 2 ist die Führungshülse 17 in nur geringfügig auf den Ansatz 25 aufgeschobener Position dargestellt, während Fig. 3 die Führungshülse 17 in bereits fast vollständig auf den Ansatz 15 aufgeschobenen Zustand wiedergibt.

Nach den Fig. 2 und 4 bis 6 weist die aus steifem Kunststoffmaterial bestehende Hülse 20 einen im wesentlichen kreiszylindrischen Querschnitt auf. Sie ist relativ dünnwandig und besitzt an ihrem hinteren Ende einen radial nach außen vorstehenden Umfangsflansch 38, durch den die Auflagefläche auf der vorderen Stirnfläche der Schraubkappe 12 vergrößert wird. Am vorderen Ende ist die Hülse 15 mit einem radial nach innen vorspringenden Stirnflansch 39 versehen, radial innerhalb von dem eine kreisförmige Nadeldurchlaßöffnung 19 vorgesehen ist, die mit dem rückwärtigen Teil 16a der Kanüle 16 ausgerichtet ist und einen etwas größeren Durchmesser als diese aufweist. Der Durchmesser der Nadeldurchlaßöffnung 19 ist jedoch deutlich kleiner als der des Schlauches 36, so daß dieser sich beim Aufschieben der Führungshülse 17 auf den Ansatz 15 am Stirnflansch 39 rund um die Nadeldurchlaßöffnung 19 abstützen kann, wie das in Fig. 3 dargestellt ist.

Der weichelastische Stopfen 21 weist eine zum Innenraum der Hülse 20 komplementäre Kreiszylinderform auf, wobei der Außendurchmesser des Stopfens 21 geringfügig größer als der Innendurchmesser der Hülse 20 ist, so daß der in die Hülse 20 von hinten eingesteckte Stopfen 21 mit seiner Mantelfläche 25 zumindest in leichtem Reibsitz mit der Hülse 20 steht. Im eingesetzten Zustand liegt der Stopfen 21 gemäß den Fig. 2 und 3 mit seiner vorderen ebenen Stirnfläche 23 an dem ebenen Stirnflansch 39 an.

Erfindungsgemäß weist der Stopfen 21 einen vorn verschlossenen und hinten offenen Axialkanal 22 auf, dessen Anordnung und Formgebung im folgenden im einzelnen beschrieben wird:

Das vordere Ende des Kanals 22 grenzt an eine unmittelbar hinter der Nadeldurchlaßöffnung 16 angeordnete Elastikplatte 18 an, die mit dem Material des weichelastischen Stopfens 21 aus einem Stück besteht und deren vordere ebene Stirnfläche 24 mit der vorderen Stirnfläche 23 des Stopfens 21 bündig ist. Die Dicke der Elastikplatte 18 ist so gewählt, daß nach dem Herausziehen des rückwärtigen Teils 16a der Kanüle 16 aus der in Fig. 3 dargestellten Durchstechposition die Durchstechöffnung sich aufgrund der elastischen Eigenschaften und der Einspannung der Elastikplatte 18 wieder von selbst schließt.

Der nach hinten offene Kanal 22 beginnt also an der hinteren Wand der Elastikplatte 18. Im vorderen Bereich $22'$ weist der Kanal 22 einen kreiszylindrischen oder geringfügig sich nach hinten erweiternden Querschnitt auf, und zwar bis zu einer Stelle deutlich hinter der Vorderkante des in endgültiger Position befindlichen Kanülenkonus 14. Der Durchmesser des Kanals 22 ist in diesem Bereich geringfügig kleiner als der Außendurchmesser der Spitze des Kanülenkonus 14, so daß im aufgesetzten Zustand des Ansatzes 15 gemäß den Fig. 2 und 3 das vordere Ende des Kanülenkonus 14 allseitig dichtend in das vordere Ende des Kanals 22 eintritt. Es wird so im Bereich der Spitze des Kanülenkonus 14 ein Dichtbereich 26 zwischen dem Kanülenkonus 14 und dem Stopfen 21 geschaffen.

Unmittelbar hinter dem Dichtbereich 26 erweitert sich der Kanal 22 sprungartig geringfügig. Ab hier nach hinten weist der Kanal 22 an seinem Umfang axiale Entlüftungsnuten 28 auf, die in den Fig. 2, 3, 5 und 6 zu erkennen sind. Beispielsweise sind drei derartige Entlüftungsnuten 28 über den Umfang verteilt angeordnet.

Ab der sprungartigen Erweiterung des Querschnittes des Kanals 22 im Anschluß an den Dichtbereich 26 besitzt der Kanal 22 bis zu seinem hinteren Ende einen gleich bleibenden Querschnitt. Dieser Querschnitt ist so dimensioniert, daß in einem bestimmten nicht zu großen Abstand hinter dem Dichtbereich 26, beispielsweise in Höhe des oberen Pfeiles der Bezugszahl 27 in Fig. 2, 3 die Außenwand des Kanülenkonus 14 in klemmenden Eingriff mit der Außenwand des Kanals 22 kommt. Aufgrund der konischen Erweiterung des Kanülenkonus 14 nach hinten wird somit die Klemmung nach hinten zwischen dem Kanülenkonus 14 und dem Stopfen 21 immer stärker. Es entsteht so ein Klemmbereich 27, wobei die Klemmkräfte so dimensioniert sind, daß hierdurch der Ansatz 15 sicher und nur durch größere von Hand aufzubringende Kräfte von dem Kanülenkonus 14 lösbar ist.

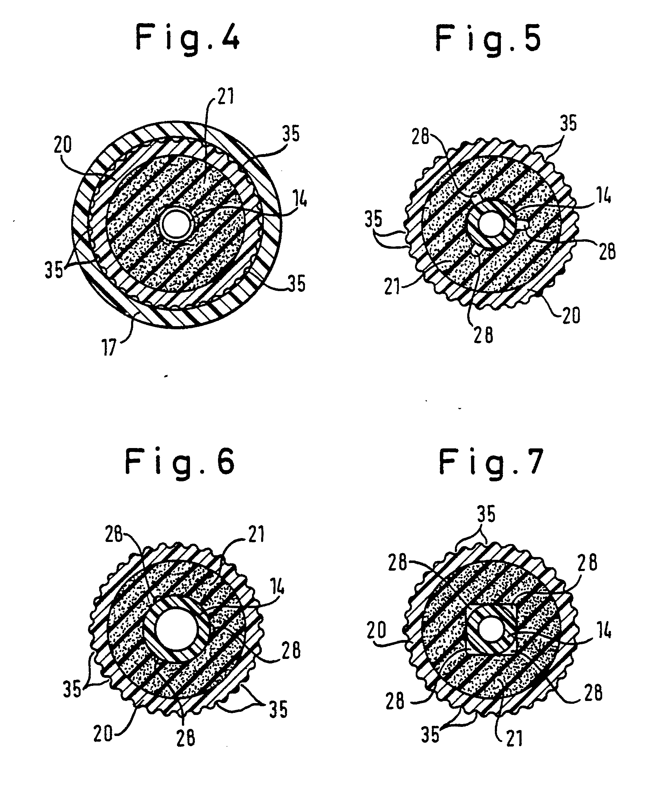

Aufgrund der nach hinten immer stärker werdenden Klemmung des Materials des Stopfens 21 werden die Entlüftungsnuten 28 nach Fig. 5 immer stärker - zusammengedrückt, wie das aus dem Schnitt der Fig. 6 hervorgeht. Die Nuten 28 müssen so tief ausgebildet sein, daß sie auch am hinteren Ende des Stopfens 21 nicht vollständig zusammengedrückt sind und noch eine axiale Entlüftung zulassen.

Im Bereich der Spitze des Kanülenkonus 14 ist in dem Stopfen 21 außerdem eine Umfangsnut 29 vorgesehen, die im Dichtbereich 26 eine definierte, jedoch nicht zu starke radiale Klemmung des Materials des Stopfens 21 am Material des Kanülenkonus 14 gewährleistet. Bevorzugt erstreckt sich die Spitze des Kanülenkonus 14 bis an die hintere Wand der Elastikplatte 18, während die Umfangsnut 29 ebenfalls unmittelbar hinter der Elastikplatte 18 beginnt.

In den Fig. 2 und 3 sind gestrichelt auch noch Bajonettverschlußmittel 30, 31 angedeutet, die beispielsweise aus einem radial von der Hülse 20 vorstehenden Zapfen 30 und einem nur angedeuteten Bajonettverschlußschlitz 31 in der Führungshülse 17 bestehen. Diese Bajonettverschlußmittel können ähnlich wie in der DE-C2 30 49 503 beschrieben ausgebildet sein.

Die Arbeitsweise der anhand der Fig. 1 bis 6 beschriebenen Blutentnahmevorrichtung ist wie folgt:

Im Herstellungsbetrieb wird zunächst der weichelastische Stopfen 21 im Reibsitz innerhalb der Hülse 20 angeordnet, wobei die Elastikplatte 18 am Stirnflansch 39 zur Anlage kommt. Anschließend wird dann der so vorgefertigte Ansatz 15 axial auf den Kanülenkonus 14 der Schraubkappe 12 aufgesetzt. Wegen der axialen Entlüftungskanäle 28 kommt es hierbei zu keinen die Abdichtung im Dichtbereich 26 oder die Klemmung im Klemmbereich 27 beeinträchtigenden Lufteinschlüssen. Während im Dichtbereich 26 mit vergleichsweise geringen radialen Klemmkräften eine vollständige Abdichtung erzielt wird, werden im Klemmbereich 27 wesentlich größere Klemmkräfte zwischen dem Stopfen 21 und dem Kanülenkonus 14 erzeugt, so daß hierdurch der Ansatz 15 sicher an der Schraubkappe 12 gehalten wird.

Nunmehr ist die Blutentnahmevorrichtung zum Zusammenwirken mit der Führungshülse 17 und der Kanüle 16 bereit.

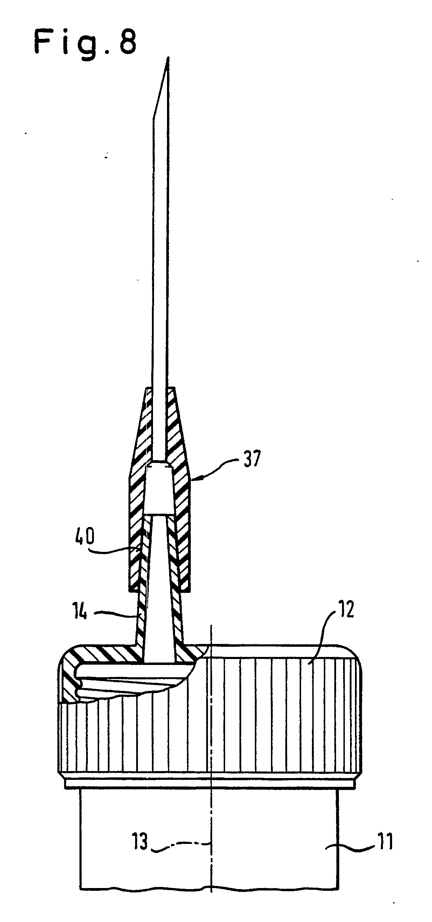

Soll - ausnahmsweise - die erfindungsgemäße Blutentnahmevorrichtung einmal mit einer normalen Kanüle verwendet werden, so wird die Hülse 20 von Hand ergriffen und unter leichtem Drehen und axialen Ziehen von dem Kanülenkonus 14 axial abgezogen. Anschließend kann dann gemäß Fig. 8 eine handelsübliche normale Kanüle 37 mit einem üblichen konischen Aufnahmeraum 40 am hinteren Ende auf den Kanülenkonus 14 aufgesetzt werden.

Nach Fig. 7 kann der Kanal 22 im Anschluß an den kreiszylindrischen Bereich 22' auch eine quadratische Form haben, wodurch die Ecken des Quadrates die axialen Entlüftungsnuten bilden, zwischen denen die durch die geraden Seiten des Quadrats gebildeten Klemmbacken vorliegen, die mit dem Außenumfang des Kanülenkonus 14 in Klemmeingriff stehen.

Ein wesentlicher Zweck der axialen Entlüftungsnuten 28 besteht auch darin, daß für den Fall, daß der Ansatz 15 nach einer Blutentnahme axial abgezogen wird, keine Flüssigkeit aus dem Inneren des Kanülenkonus 14 nach außen gesaugt wird. Da nämlich der Dichtbereich 26 axial nur sehr kurz ausgebildet ist, kommt der Bereich 22' des Kanals schon nach einer geringen Abzugsbewegung der Hülse 21 mit den Entlüftungsnu ten 28 in Strömungsverbindung, wodurch der Aufbau eines das Ansaugen von Flüssigkeit ermöglichenden Unterdrucks im Kanalbereich 22' wirksam vermieden wird.

## Ansprüche

1. Blutentnahmevorrichtung mit einem einseitig offenen, evakuierten bzw. evakuierbaren Entnahmerohr (11), auf das eine Verschlußkappe (12) lösbar aufsetzbar ist, welche einen parallel zur Rohrachse (13) nach vorn vorstehenden Kanülenkonus (14) trägt, der sich vorzugsweise konzentrisch in einem einen kleineren Durchmesser als das Entnahmerohr (11) aufweisenden, zumindest im wesentlichen zylindrischen, im wesentlichen als nach unten offenes Rohr ausgebildeten Ansatz (15) hineinerstreckt, der aus einer nach hinten offenen, nach vorne bis auf eine Nadeldurchlaßöffnung (19) geschlossenen steifen Hülse (20) und einem dicht auf den Kanülenkonus (14) aufsetzbaren, einen Kanal (22) aufweisenden Gegenstück (21) besteht und auf den eine im wesentlichen dazu komplementäre, hinten offene und vorne eine beidseitig angeschärfte Kanüle (16) vorzugsweise ebenfalls konzentrisch tragende Führungshülse (17) axial von vorne aufschiebbar ist, wobei das sich in das Innere der Führungshülse (17) erstreckende Kanülenende (16a) eine nahe der Nadeldurchlaßöffnung (19) angeordnete, in axialer Ausrichtung mit diesem befindliche sowie einen Bestandteil des Ansatzes (15) darstellende Elastikplatte (18) durchsticht und so eine Strömungsverbindung zwischen dem Inneren der Kanüle (16) und dem Inneren des Kanülenkonus (14) herstellt, dadurch **gekennzeichnet,** daß das Gegenstück ein in die steife Hülse (20) passend eingesetzter, weichelastischer Stopfen (21), der vorzugsweise aus Gummi be steht, ist, welcher einen zur Schraubkappe (12) hin offenen, mit dem Kanülenkonus (14) in einen den Ansatz (15) fest, jedoch lösbar an der Schraubkappe (14) haltenden Klemmsitz bringbaren Kanal (22) aufweist, der vorn durch die Elastikplatte (18) verschlossen ist.

2. Vorrichtung nach Anspruch 1, dadurch **gekennzeichnet,** daß die Elastikplatte (18) mit dem Stopfen (21) aus einem Stück besteht und/oder daß die vordere Stirnfläche (24) der Elastikplatte (18) mit der vorderen Stirnfläche (23) des Stopfens (21) bündig ist.

3. Vorrichtung nach einem der vorhergehenden Ansprüche, dadurch **gekennzeichnet,** daß der Stopfen (21) eine kreiszylindrische Mantelfläche (25) und die Hülse (20) eine kreiszylindrische Innenumfangsfläche aufweist.

4. Vorrichtung nach einem der vorhergehenden Ansprüche, dadurch **gekennzeichnet,** daß der Stopfen (21) zumindest bei aufgesetztem Ansatz (15) mit seiner Mantelfläche (25) gegen die Innenumfangsfläche der Hülse (20) gepreßt wird, wobei insbesondere der Stopfen (21) ohne eingesteckten Kanülenkonus (14) im leichten, selbsthaltenden Reibsitz in der Hülse (20) steckt.

5. Vorrichtung nach einem der vorhergehenden Ansprüche, dadurch **gekennzeichnet,** daß der Kanal (22) im Bereich der Spitze des Kanülenkonus (14) dichtend vom Material des Stopfens (21) umgeben ist, so daß dort ein Dichtbereich (26) vorliegt, wobei insbesondere der Kanal (22) hinter dem Dichtbereich (26) zumindest eine axial verlaufende Entlüftungsnut (28) aufweist und in diesem Bereich (27) in festem Klemmsitz mit dem Kanülenkonus (14) lösbar verbunden ist, und daß zweckmäßigerweise sich der Dichtbereich (26) über 5 bis 20 %, insbesondere etwa 10 % der Länge des mit dem Kanülenkonus (14) in Kontakt stehenden Teils des Kanals (22) erstreckt.

6. Vorrichtung nach einem der vorhergehenden Ansprüche, dadurch **gekennzeichnet,** daß der Kanal (22) hinter der Elastikplatte (18) einen kurzen Abschnitt (22') runden Querschnitts aufweist, der einen geringfügig kleineren Durchmesser als der vordere Endbereich des ebenfalls einen kreisrunden Querschnitt aufweisenden Kanülenkonus (14) besitzt und in den der vordere Endbereich des Kanülenkonus (14) dichtend eingreift und/oder daß der Querschnitt des Kanals (22) sich hinter dem Dichtbereich (26) erweitert, jedoch nur so geringfügig, daß aufgrund der konischen Erweiterung des Kanülenkonus (14) noch ein Klemmbereich (27) vorliegt, wobei insbesondere sich der Querschnitt des Kanals (22) hinter dem Dichtbereich (26) sprungartig erweitert und dann bis zum hinteren Ende des Kanals (22) unverändert bleibt.

7. Vorrichtung nach einem der vorhergehenden Ansprüche, dadurch **gekennzeichnet,** daß im Klemmbereich (27) wenigstens eine, vorzugsweise jedoch mehrere über den Umfang verteilte axiale Entlüftungsnuten (28) von solcher Tiefe vorgesehen sind, daß auch bei auf dem Kanülenkonus (14) aufgesetztem Ansatz noch ein Luftdurchlaß am hinteren Ende verbleibt oder daß der Kanal (22) im Klemmbereich (27) einen unrunden, vorzugsweise quadratischen Querschnitt aufweist.

8. Vorrichtung nach einem der vorhergehenden Ansprüche, dadurch **gekennzeichnet,** daß sich im Bereich des vorderen Endes des Kanülenkonus (14) in der Mantelfläche (25) des Stopfens (22) eine Umfangsnut (29) befin det, welche ein sanftes und gleichmäßiges federndes Aufweiten des Kanals (22) beim Einstecken der Spitze des Kanülenkonus (14) in diesem Bereich und somit ein gleichmäßiges dichtes Anliegen des Stopfens (22) im Dichtbereich (26) am Umfang des Kanülenkonus (14) gestattet.

9. Vorrichtung nach einem der vorhergehenden Ansprüche, dadurch **gekennzeichnet,** daß die Spitze des Kanülenkonus (14) zumindest bis annähernd an die hintere Fläche der Elastikplatte (18) reicht.

10. Vorrichtung nach Anspruch 15 und 16, dadurch **gekennzeichnet,** daß die Umfangsnut (26) unmittelbar hinter dem Bereich der Elastikplatte (18) beginnt und/oder daß die Hülse (20) und die Führungshülse (17) Bajonettverschlußmittel (30, 31) aufweisen.

Fig.

# Fig.2

# Fig.3

Fig.4

Fig.5

Fig.6

Fig.7

# Fig.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-C-2948653 (WALTER SARSTEDT KUNSTSTOFF-SPRITZGUSSWERK) <br> * das ganze Dokument * <br> --- | 1-9 | A61B5/14 <br> A61M5/32 |
| D,A | DE-C-3049503 (WALTER SARSTEDT KUNSTSTOFF-SPRITZGUSSWERK) <br> * das ganze Dokument * <br> --- | 1, 10 | |
| A | US-A-3994295 (G.L.WULFF) <br> * Zusammenfassung; Figuren 1-4 * <br> --- | 1 | |
| A | EP-A-0047176 (WARNER-LAMBERT COMPANY) <br> * Zusammenfassung; Figuren 1, 2 * <br> ----- | 1 | |

RECHERCHIERTE SACHGEBIETE (Int. Cl.5)

A61B
A61M

Der vorliegende Recherchenbericht wurde für alle Patentansprüche erstellt

| Recherchenort | Abschlußdatum der Recherche | Prüfer |
|---|---|---|
| DEN HAAG | 19 JULI 1990 | HUNT B.W. |